Europäisches Patentamt

European Patent Office

Office européen des brevets

(11) Veröffentlichungsnummer: **0 374 502**
**A2**

## (12) EUROPÄISCHE PATENTANMELDUNG

(21) Anmeldenummer: **89121578.2**

(22) Anmeldetag: **21.11.89**

(51) Int. Cl.5: **A61M 21/00**

(30) Priorität: **23.12.88 DE 3843720**

(43) Veröffentlichungstag der Anmeldung:
**27.06.90 Patentblatt 90/26**

(84) Benannte Vertragsstaaten:
**AT BE CH DE ES FR GB IT LI NL SE**

(71) Anmelder: **Hellmann, W. Reiner**
**Montsalvatstrasse 7**
**D-8000 München 40(DE)**

(72) Erfinder: **Hellmann, W. Reiner**
**Montsalvatstrasse 7**
**D-8000 München 40(DE)**

(74) Vertreter: **MEISSNER, BOLTE & PARTNER**
**Widenmayerstrasse 48 Postfach 860624**
**D-8000 München 86(DE)**

(54) Anordnung zur Durchführung psychotherapeutischer Behandlungen.

(57) Die Durchführung therapeutischer Behandlungen zur Bekämpfung psychischer oder psychosomatischer Erkrankungen ist sowohl für den Behandelnden als auch für den Behandelten sehr zeitaufwendig. Es wird eine Anordnung zur Durchführung psychotherapeutischer Behandlungen vorgeschlagen, die ein Zusatzgerät für einen Fernsehempfänger umfaßt, um den Zuschauer während des Betrachtens von Fernsehsendungen zu beeinflussen. Hierbei ist eine erste Schaltungsanordnung vorgesehen, um zeitweilig von einem Sender kommende Bildsignale gegen gespeicherte Bildsignale auszutauschen. Der Austausch erfolgt derart, daß die eingeschobenen Bilder die gewünschte Beeinflussung beim Zuschauer hervorrufen, andererseits aber die vom Sender ausgestrahlten Informationen im wesentlichen ungestört empfangen werden können. Auf diese Weise kann die Behandlung in die Freizeit verlegt werden. Darüber hinaus bekommt der Behandelnde eine positive Einstellung gegenüber der Therapie.

FIG.1

## Anordnung zur Durchführung psychotherapeutischer Behandlungen

Die Erfindung betrifft eine Anordnung zur Durchführung psychotherapeutischer Behandlungen nach dem Oberbegriff des Patentanspruches 1.

Eine Vielzahl von psychischen oder psychosomatischen Erkrankungen läßt sich durch psychotherapeutische Behandlungen zumindest in ihren Auswirkungen abschwächen oder sogar heilen. So z.B. lassen sich viele Erfolge bei der Behandlung von Alkoholismus oder anderen Suchterkrankungen ebenso nachweisen wie bei der Behandlung von Schlafstörungen, Störungen des vegetativen Nervensystems usw.. Ein wesentlicher Punkt liegt bei derartigen Behandlungen darin, daß der Behandelte regelmäßig zu den Sitzungen erscheint, was natürlich mit einem hohen Zeitaufwand sowohl für den Behandelnden als auch für den Behandelten verbunden ist.

Aufgabe der Erfindung ist es, eine Anordnung zur Durchführung psychotherapeutischer Behandlungen aufzuzeigen, durch welche die Behandlung insbesondere hinsichtlich des dafür notwendigen Zeitaufwandes erleichtert wird.

Diese Aufgabe wird durch eine Anordnung der im Patentanspruch 1 beschriebenen Art gelöst.

Ein wesentlicher Punkt der Erfindung liegt darin, daß die Behandlung in die "Freizeit" verlegt wird, ohne aber diese Freizeit dabei einzuschränken. Insbesondere dreht es sich bei der therapeutischen Beeinflussung darum, daß auf das "Hintergrundsbewußtsein" bzw. auf das Unterbewußtsein einwirkende Informationen bei der Betrachtung von Fernsehsendungen vom Zuschauer aufgenommen werden, die so ausgewählt sind, daß eine Beeinflussung in der gewünschten Richtung stattfindet. Wenn z.B. eine Behandlung von Alkohol- oder Nikotinabhängigkeit durchgeführt werden oder auch eine übermäßige Freßsucht bekämpft werden soll, so ist die Beeinflussung durch Bilder oder Bildfolgen möglich, die eine derartige unterbewußte Beeinflussung durch Erzeugung eines Widerwillens gegen Alkohol, Nikotin oder Nahrungsmittel erzeugt, wie sie aus der Literatur an sich bekannt sind. Soll Schlaflosigkeit behandelt werden, so eignet sich die Einspielung von entsprechend anderen Bildern oder Bildfolgen mit einschläfernder Wirkung.

Weiterhin ist beim Erfindungsgedanken wesentlich, daß der Behandelte sich als ganz normaler Fernsehzuschauer fühlt und das betrachtete Programm genießen kann, so daß er eine positive Einstellung zu der an ihm vorgenommenen Behandlung bekommt.

Ein weiterer Vorteil der Erfindung liegt darin, daß die eingangs genannten Suchterkrankungen oftmals gerade durch das Betrachten von Fernsehsendungen, auf Videorekorder aufgezeichneten Filmen oder dergleichen verstärkt werden. Gerade dabei entwickeln anfällige Menschen oftmals für sie selbst schädliche Verhaltensweisen, konsumieren Alkohol, Gebäck, "Knabbersachen" und dergleichen Kleinigkeiten und rauchen auch mehr. Insbesondere dann, wenn sich die betreffende Person auf die betrachtete Sendung konzentriert, verliert sie leicht die Kontrolle über diese Verhaltensweisen.

Bei Kindern und Jugendlichen spielt oftmals die "Fernsehsucht" selbst, also das unkontrollierte Konsumieren von Unterhaltung, die Hauptrolle bei der Verursachung feststellbarer Schäden, die dann insbesondere in mangelnder Konzentrationsfähigkeit, Schlaflosigkeit, Nervosität usw. resultieren. Will man den Zuschauer, insbesondere Kinder und Jugendliche, vom übermäßigen Unterhaltungskonsum abbringen, so eignet sich die Einspielung von einschläfernden bzw. das zu-Bett-gehen attraktiv machenden Bildern oder Bildfolgen, wobei diese vorzugsweise nicht immer eingeblendet werden, sondern nur dann, wenn entweder eine bestimmte Tages- bzw. Nachtzeit überschritten oder aber die Fernsehdauer insgesamt eine bestimmte Länge erreicht hat.

Wichtig ist zu erwähnen, daß für die Behandlung bestimmter Erkrankungen oder Schädigungen bzw. für eine entsprechende Vorbeugung "konfektionierte" Bilder oder Bildfolgen speicherbar sind, so daß in vielen Fällen der Therapeut oder sogar der Behandelte selbst, der um seine Schwierigkeiten weiß, einen entsprechend vorgefertigten bzw. programmierten Bildspeicher verwenden kann. Dies senkt die Kosten.

Dadurch, daß die im Bildspeicher gespeicherten Informationen synchron zum Fernsehbild eingespielt werden, ergibt sich keine störende Wirkung auf die Fernsehübertragung selbst. Der Bruchteil, zu welchem die vom Sender gesendeten Bildinformationen durch gespeicherte Bildinformationen ersetzt werden sollen, wird vorzugsweise so gewählt, daß keine Störung der Haupt-Informationen auftritt. Dies ist besonders dadurch zu erreichen, daß die empfangenen Bilder in Zeitabständen gegen gespeicherte Bilder austauschbar sind, welche nach einer Zufallsverteilung zwischen einer Mindest- und einer Maximalfrequenz schwanken. Dadurch wird der "Austauschrhythmus nicht mehr störend erkennbar. Dies kann weiterhin dadurch verstärkt werden, daß die empfangenen Bilder nur halbbildweise gegen die gespeicherten Bilder ausgetauscht werden.

Man kann das Zusatzgerät direkt in den Fernsehempfänger unter Auftrennung des Bild-Kanals einbauen, jedoch ist es von Vorteil, wenn das Zusatzgerät als selbständiges Gerät ausgeführt ist. Hierbei ist es von besonderem Vorteil, wenn die Toninformation nicht angetastet, sondern "ungestört" übertragen wird, da das menschliche Ohr gegenüber derartigen Unterbrechungen sehr empfindlich ist.

Bei einer bevorzugten Ausführungsform der Erfindung ist der Bildspeicher austauschbar ausgebildet, so daß der Benutzer bzw. sein Therapeut oder Erzieher das gewünschte bzw. notwendige Bildmaterial in Form eines Modules auswechseln kann.

Weitere bevorzugte Ausführungsformen der Erfindung ergeben sich aus den Unteransprüchen und der nachfolgenden Beschreibung der Erfindung, die anhand von Abbildungen näher erläutert wird. Hierbei zeigen:

Fig. 1 ein Blockschaltbild einer ersten Ausführungsform der Erfindung, wobei das Zusatzgerät in den Fernsehempfänger eingebaut ist;

Fig. 2 ein Blockschaltbild einer Ausführungsform der Erfindung, bei welcher das Zusatzgerät vor dem Fernsehgerät angeordnet ist;

Fig. 3 ein detaillierteres Blockschaltbild der Ausführunsform nach Fig. 2;

Fig. 4 einen Einzelblock aus Fig. 3;

Fig. 5 eine weitere Ausführungsform der Erfindung, ähnlich der nach Fig. 3; und

Fig. 6 einen Einzelblock der Anordnung nach Fig. 5 in detaillierterer Darstellung.

Bei der folgenden Beschreibung der Erfindung wird der Einfachheit halber lediglich auf Schwarz-Weiß-Empfänger Bezug genommen. Selbstverständlich wird das hier aufgezeigte Zusatzgerät vorzugsweise zur Verbindung mit Farbfernsehgeräten ausgebildet, wobei jedoch die einzuspielende Bildinformation zur Verminderung des Schaltungsaufwandes als Schwarz-Weiß-Information gespeichert und gesendet wird.

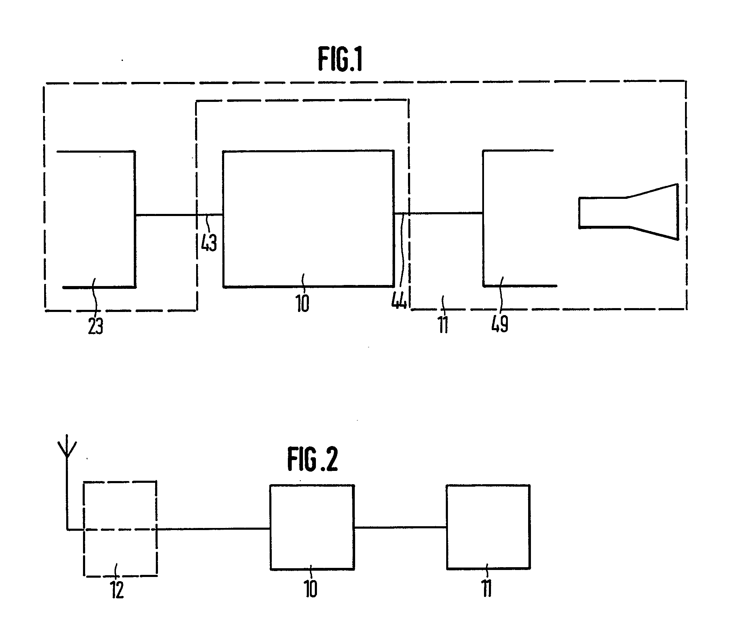

In Fig. 1 ist ein Fernsehempfänger 11 mit Umrißlinien schematisiert dargestellt, der einen dem (nicht gezeigten) Tuner und ZF-Verstärker nachgeordneten Videodemodulator 23 aufweist, der ein BAS-Signal an eine Videovorstufe 46 übermittelt, der dann wiederum die üblichen weiteren Baugruppen nachgeschaltet sind. Die Verbindung zwischen dem Videodemodulator 23 und der Videovorstufe 49 wird aufgetrennt, so daß das Zusatzgerät 10 mit seinem BAS-Eingang 43 und seinem BAS-Ausgang 44 in die Anordnung eingekoppelt werden kann. Das Zusatzgerät 10 wird weiter unten näher beschrieben.

Bei der in Fig. 2 gezeigten Ausführungsform der Erfindung ist das Zusatzgerät 10 gesondert vom Fernsehempfänger 11 angeordnet und zwar zwischen der Antenne und dem Fernsehempfänger 11 bzw. zwischen dem Ausgang eines Videorekorders 12 und dem Fernsehempfänger 11.

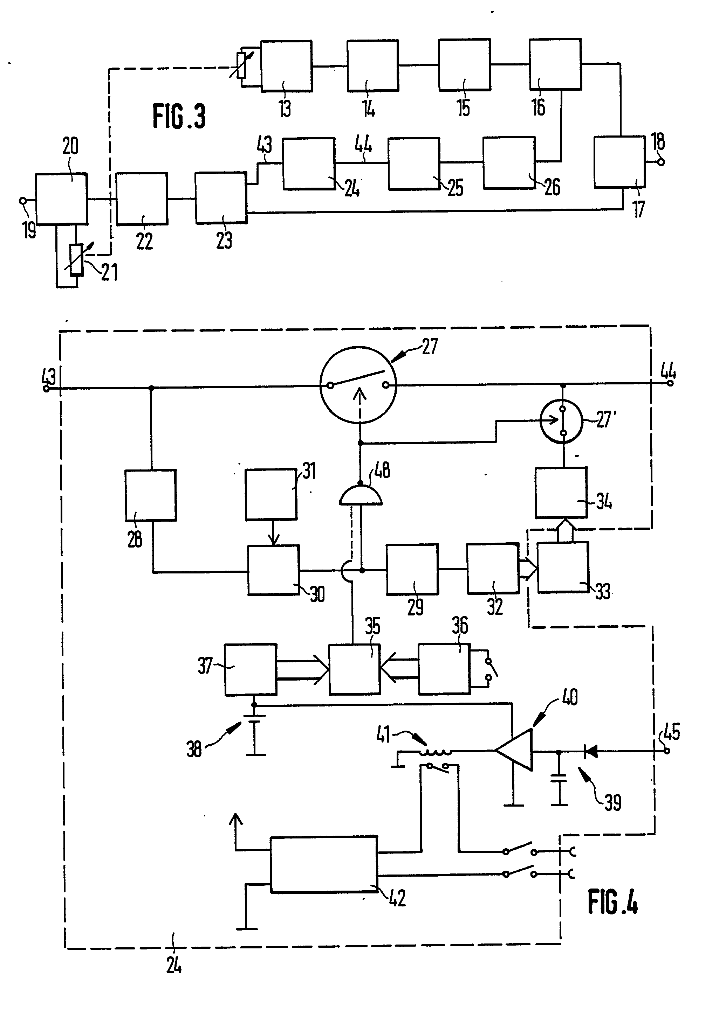

Im folgenden wird anhand der Figuren 3 und 4 eine erste bevorzugte Ausführungsform des Zusatzgerätes 10 näher beschrieben, wobei das Blockschaltbild nach Fig. 3 ein separat aufstellbares Gerät (siehe Fig. 2) betrifft, während Fig. 4 einen Kernteil des Zusatzgerätes zeigt, der zusammen mit entsprechenden, nicht gezeigten Stromversorgungseinrichtungen usw. als Zusatzgerät in einen Fernsehempfänger einbaubar ist (siehe Fig. 1).

Wie in Fig. 3 gezeigt, umfaßt das Zusatzgerät 10 einen Tuner 20, der über eine Abstimmeinheit 21 auf einen gewünschten Sender abstimmbar und über einen HF-Eingang 19 an diesen ankoppelbar ist. Diese Ausführungsform der Erfindung eignet sich besonders zur Einkoppelung zwischen einem Videorekorder 12 und einem Fernsehempfänger 11 (siehe Fig. 2), wobei dann die Abstimmeinrichtung 21 auf die Ausgangs-Frequenz des Tuners abgestimmt wird.

Dem Tuner 20 nachgeschaltet ist eine ZF-Vorstufe, der ein Videodemodulator 23 folgt. Der BAS-Ausgang des Demodulators 23 liegt auf dem BAS-Eingang einer BAS-Verarbeitungsschaltung 24, an deren Ausgang 44 ein Entzerrer 25 mit seinem Eingang liegt. Die Ausgangssignale des Entzerrers 25 werden über einen Modulationsverstärker 26 auf den Eingang einer Endstufe 16 geführt, die von einem Oszillator 13 über einen zwischengeschalteten Vervielfacher 14 und eine Vorstufe 15 gespeist wird. Das Ausgangssignal der Endstufe 16 geht auf den Bild-Eingang einer Bild-/Tonweiche 17, deren Toneingang auf den Ton-Ausgang des Videodemodulators 23 geführt ist. Am Ausgang 18 steht somit wieder ein HF-Signal, das auf den Antenneneingang eines nachgeschalteten Fernsehempfängers 11 geführt werden kann.

Die BAS-Verarbeitungsschaltung 24 weist einen steuerbaren Umschalter 27, 27' auf, der in seiner einen Stellung den BAS-Eingang 43 mit dem BAS-Ausgang 44 verbindet, in seiner anderen Stellung diese Verbindung unterbricht und den Ausgang eines Bildgenerators 34 mit dem BAS-Ausgang 44 verbindet.

Am BAS-Eingang 43 liegt weiterhin der Eingang einer Amplitudensiebschaltung 28, die derart ausgebildet ist, daß zu Beginn eines jeden Einzelbild-Signales ein Ausgangspuls an ihrem Ausgang abgegeben und einem Teiler 30 zugeführt wird. Der Teiler 30 ist über eine Voreinstellschaltung 31 in seinem Teilverhältnis einstellbar, so daß bei einem Bruchteil von Bildsignalen, die am BAS-Eingang 43 anliegen, am Ausgang des Teilers 30 ein Bildanfangspuls entsteht. Dieser Bildanfangspuls wird einerseits über ein NAND-Gatter 48 auf den Steuereingang des Schalters 27 geführt, andererseits liegt das Signal auf dem Steuereingang einer Synchronisierschaltung 29. Die Synchronisierschaltung 29 ist derart ausgebildet, daß auf jeden Synchronisierpuls an ihrem Eingang hin eine Folge von Einzelpulsen ausgegeben wird, deren zeitlicher Abstand zueinander dem zeitlichen Abstand einzelner Pixel im Bild entspricht. Diese Pulsfolge wird dem Eingang eines Adressengenerators 32 zugeführt, der einen Speicher 33 entsprechend anwählt. Im Speicher 33 ist ein Einzelbild oder eine Folge von Einzelbildern gespeichert, die über das Adressensignal aus dem Adressengenerator 32 pixelweise ausgelesen und an einen Bildgenerator 34 übermittelt werden. Der Bildgenerator 34 übersetzt die erhaltenen Informationen in Analogsignale, die dann auf den BAS-Ausgang 44 geführt werden.

Bei der so beschriebenen Anordnung wird somit eine durch die Einstellung des Teilers 30 bzw. der Voreinstellschaltung 31 definierte Anzahl von Bildern, die am BAS-Eingang 43 ankommen durch Öffnen des Schalters 47 abgetrennt und durch Bilder ersetzt, die im Speicher 33 gespeichert sind. Das Ersetzen erfolgt hierbei bildgenau, so daß ein flimmerfreies Einspielen sichergestellt wird. Bei einer bevor zugten Ausführungsform der Erfindung umfaßt die Voreinstellschaltung 31 einen Pseudo-Zufallsgenerator, welcher derart ausgebildet ist, daß der Teiler 30 mit zufällig wechselnden Teilerverhältnissen programmiert wird, die sich zwischen einer Minimal- und einer Maximal-Teilerzahl bewegen. Die Mindest-Teilerzahl ist hierbei so bemessen, daß zur Beeinflussung hinreichend oft Bilder aus dem Speicher 33 eingesetzt werden, andererseits aber der Schalter 27 nur so oft geöffnet wird, daß keine merkbare Beeinträchtigung der Bildinformation auftritt, die der Zuschauer eigentlich sehen will.

In Fig. 4 ist eine Zusatzeinrichtung dargestellt, die einen Komparator 35 umfaßt, dessen einer Vergleichseingang mit dem Ausgang eines Zeiteinstellschalters 36 und dessen anderer Vergleichseingang mit dem Ausgang einer Echtzeituhr 37 verbunden ist, die von einer Batterie 38 ständig gespeist wird. Der Komparator 35 gibt dann ein Ausgangssignal ab, wenn die im Zeiteinstellschalter 36 eingestellte Uhrzeit mit der tatsächlichen Uhrzeit übereinstimmt, die aus der Echtzeituhr 37 ausgelesen wird. Dieses Ausgangssignal wird auf einen zweiten Eingang des NAND-Gatters 48 geführt (unterbrochene Linie in Fig. 4). Bei dieser bevorzugten Ausführungsform der Erfindung wird also nur dann der Schalter 27 zum Einspielen von Bildern aus dem Speicher 33 geöffnet, wenn die tatsächliche Uhrzeit eine voreingestellte Uhrzeit überschreitet. Hierbei ist darauf hinzuweisen, daß bei der in Fig. 4 gezeigten Darstellung der Übersichtlichkeit halber die (an sich vorgesehene) Einrichtung zum Abtrennen des Ausganges des Bildgenerators 34 vom BAS-Ausgang 44 nicht dargestellt ist, die dann zur Abkoppelung wirksam wird, wenn der Komparator 35 kein Ausgangssignal abgibt.

Gemäß einer weiteren bevorzugten Ausführungsform der Erfindung ist in der BAS-Verarbeitungsschaltung 24 eine Stromversorgungseinrichtung vorgesehen, die mit einem Ton

Eingang 45 auf einer Lautsprecherausgangsbuchse des Fernsehgerätes 11 liegt. Die vom Toneingang 45 kommenden Tonsignale werden über einen Tiefpaß-Gleichrichter 39 in einen Gleichstrompegel verwandelt, der auf dem Eingang eines Schwellenschalters 40 liegt, welcher von der Batterie 38 ständig mit Strom versorgt wird. Der Schwellenschalter 40 steuert ein Relais 41, das dem einen Netz-Eingangspol einer Stromversorgungsschaltung 42 vorgeschaltet ist, die das Zusatzgerät insgesamt mit Strom versorgt. Durch diese Anordnung ist gewährleistet, daß die Haupt-Stromversorgung des Zusatzgerätes nur dann eingeschaltet wird, wenn aus dem Fernsehgerät irgendein Tonsignal kommt. Die Empfindlichkeit des Schwellenschalters 40 bzw. die Zeitkonstante des Tiefpaß-Gleichrichters 39 ist hierbei so gewählt, daß der beim Einschalten des Fernsehgerätes 11 am Lautsprecherausgang abgreifbare Impuls zu einem Einschalten des Relais 44 führt und daß dieses einige Minuten lang im eingeschalteten Zustand gehalten wird.

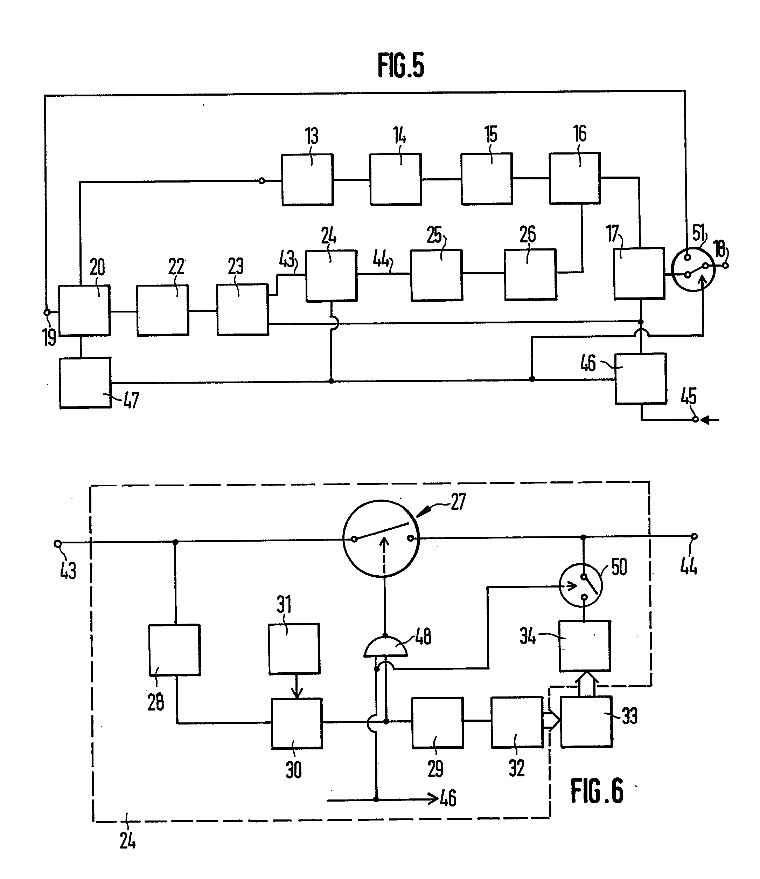

Im folgenden wird eine weitere bevorzugte Ausführungsform der Erfindung anhand der Figuren 5 und 6 näher beschrieben. Diese Ausführungsform der Erfindung unterscheidet sich von der zuvor beschriebenen Ausführungsform in erster Linie dadurch, daß Einrichtungen zum selbsttätigen Abgleich des Tuners 20 auf einen Sender vorgesehen sind, der am Fernsehgerät 11 eingestellt wird. Diese Einrichtungen umfassen einen Ton-Komparator 46, an dessen einem Eingang das Ton-Ausgangssignal aus dem Videodemodulator 23 und an dessen anderem Vergleichereingang das Ton-Ausgangssignal aus dem Fernsehempfänger anliegen. Der Ausgang des Ton-Komparators 46 liegt auf einem Steuereingang einer Suchlaufschaltung 47. Diese ist so ausgebildet, daß der Tuner 20 solange hinsichtlich seiner Empfangsfrequenz verstimmt wird, bis das im Zusatzgerät bzw. von dessen Tuner 20 empfangene Signal mit demjenigen übereinstimmt, das aus dem Fernsehgerät 11 kommt. Der Vergleich wird bei dieser Ausführungsform der Erfindung anhand der Tonsignale vorgenommen, da diese bei den allermeisten Fernsehempfängern 11 über eine Lautsprecherbuchse abgreifbar sind. Selbstverständlich ist es hier möglich, auch das Bildsignal zu Vergleichszwecken heranzuziehen.

Wie aus Fig. 6 hervorgeht, wird das Signal aus dem Tonkomparator 46 weiterhin noch einem weiteren Eingang des NAND-Gatters 48 sowie einem Steuereingang eines Schalters 50 zwischen dem Ausgang des Bildgenerators 34 und dem BAS-Ausgang 44 zugeführt. Dadurch wird sichergestellt, daß eine Bildeinblendung nur dann erfolgt, wenn der Tuner 20 auf die im Fernsehgerät 11 eingestellte Empfangsfrequenz abgestimmt ist. Weiterhin ist der Ausgang des Tonkomparators 46 auf den Steuereingang eines Umschalters 51 geführt, dessen einer Eingang auf dem Ausgang der Bild-/Tonweiche 17 und dessen anderer Eingang auf dem HF-Eingang 19 liegt, während sein Ausgang den HF-Ausgang 18 bildet. Dieser Schalter 51 ist so ausgebildet, daß bei Nicht-Übereinstimmung der dem Komparator 46 zugeführten Tonsignale die Punkte 18 und 19 fest verbunden sind und nur bei Übereinstimmung der beiden Tonsignale der Ausgang der Bild-/Tonweiche 17 dem nachgeschalteten Fernsehempfänger 11 zugeführt wird.

Bei einer weiteren, hier nicht im einzelnen gezeichneten bevorzugten Ausführungsform der Erfindung ist eine Vielzahl von Schaltungsanordnungen gemäß Fig. 3 in Parallelschaltung vorgesehen, wobei die HF-Eingänge 19 direkt gekoppelt und die HF-Ausgänge 18 über Mischerschaltungen verbunden sind und wobei jede Einzelschaltung über ihre Abstimmeinrichtungen 21 auf einen anderen, am Aufstellort empfangbaren Sender abgestimmt ist. Eine derartige Anordnung kann somit in die Wohnungs-Antennenanlage eingebaut werden. Durch entsprechend in den einzelnen Anordnungen vorzusehende Verzögerungseinrichtungen können dann alle (verschiedenen) BAS-Signale synchronisiert werden. Auf diese Weise muß zwar in jeder der Einzelanordnungen ein Schalter gemäß Fig.4 oder 6, jedoch nur eine einzige Bildeinspeisvorrichtung (Blöcke 28-34; 48) vorgesehen werden.

An dieser Stelle wird ausdrücklich darauf hingewiesen, daß die zuvor beschriebenen Einzel-Baugruppen miteinander kombiniert werden können, ohne dabei den Rahmen der Erfindung zu verlassen. Weiterhin ist es auch möglich, die beschriebene Anordnung in einen Videorekorder oder dergleichen weiteres Zusatzgerät einzubauen.

## Ansprüche

1. Anordnung zur Durchführung psychotherapeutischer Behandlungen,
**gekennzeichnet durch**
ein Zusatzgerät für einen Fernsehempfänger, zur Beeinflussung des Zuschauers beim Betrachten von Fernsehsendungen, umfassend
eine erste Schaltanordnung (27), zum zeitweiligen Abschalten des von einem Sender, wie Fernsehsender, Videorekorder oder dergleichen gesendeten Bildsignales während des Empfangs einzelner Bilder und zum Umschalten des Fernsehempfängers (11) auf Ausgangssignale eines Bildspeichers (33), in welchem mindestens ein erkennbares Bild repräsentierende Informationen gespeichert sind, und eine mit dem Bildsignal synchronisierbare Steuerschaltung (28-31) zum Steuern der Schaltanordnung (27), die derart ausgebildet ist, daß ein Bruchteil der vom Sender gesendeten Bildinformationen durch gespeicherte Bildinformationen ersetzbar ist.

2. Anordnung nach Anspruch 1,
**dadurch gekennzeichnet,**
daß die Steuerschaltung (28-31) derart ausgebildet ist, daß die empfangenen Bilder in einer zufälligen Folge gegen gespeicherte Bilder austauschbar sind.

3. Anordnung nach einem der vorhergehenden Ansprüche,
**dadurch gekennzeichnet,**
daß die Steuerschaltung (28-31) derart ausgebildet ist, daß die empfangenen Bilder halbbildweise gegen gespeicherte Bilder austauschbar sind.

4. Anordnung nach einem der vorhergehenden Ansprüche,
**dadurch gekennzeichnet,**
daß eine Umgehungsweiche (17) zur kontinuierlichen Übertragung des Tonsignales vorgesehen ist.

5. Anordnung nach einem der vorhergehenden Ansprüche,
**dadurch gekennzeichnet,**
daß die Steuerschaltung eine Echtzeituhr (37) und eine von Hand betätigbare Programmiereinrichtung (35, 36) aufweist, die derart ausgebildet ist, daß die gespeicherten Bildinformationen mit einer vorprogrammierten Uhrzeit beginnend gegen gesendete Bilder austauschbar sind.

6. Anordnung nach einem der vorhergehenden Ansprüche,
**dadurch gekennzeichnet,**
daß der Bildspeicher (33) derart ausgebildet und mit einem Adressengenerator (32) verbunden ist, daß eine Vielzahl von Einzelbildern speicherbar und in vorbestimmter Reihenfolge abrufbar ist.

7. Anordnung nach einem der vorhergehenden Ansprüche,
**dadurch gekennzeichnet,**
daß der Bildspeicher (33) über Steckverbindereinrichtungen austauschbar angeordnet ist.

8. Anordnung nach einem der vorhergehenden Ansprüche,
**dadurch gekennzeichnet,**
daß der Bildspeicher (33) einen Halbleiterspeicher umfaßt.

9. Anordnung nach einem der vorhergehenden Ansprüche,
**dadurch gekennzeichnet,**
daß ein selbstabstimmbarer Empfänger (20, 47), eine Vergleichereinrichtung (46) und eine an den Fernsehempfänger (11) ankoppelbare Bild- und/oder Tonabtasteinrichtung (45) vorgesehen sind, die derart ausgebildet sind, daß der Empfänger (20, 47) zur Durchführung eines Suchlaufes ansteuerbar und dann auf der momentan eingestellten Empfangsfrequenz fixierbar ist, wenn das vom Empfänger (20, 47) empfangene Bild-/Tonsignal mit demjenigen aus dem Fernsehempfänger (11) übereinstimmt.

10. Anordnung nach einem der vorhergehenden Ansprüche,
**dadurch gekennzeichnet,**
daß eine Detektoreinrichtung (39, 40) vorgesehen ist, die derart ausgebildet und angeordnet ist, daß eine Stromversorgung (42) zur Energieversorgung des Zusatzgerätes dann einschaltbar ist, wenn der Fernsehempfänger (11) eingeschaltet ist.

11. Anordnung nach einem der Ansprüche 1 bis 8,
**dadurch gekennzeichnet,**
daß eine Vielzahl von Baugruppen vorgesehen ist, die jeweils eine auf einen Sender abstimmbare HF-Empfangseinheit (19-23) zur Erzeugung eines BAS-Signales, eine gleichlaufend abstimmbare HF-Sendeeinheit (13-17; 25, 26) zur Umsetzung des BAS-Signales in ein HF-Ausgangssignal, eine Vielzahl der ersten Schaltanordnungen (27) zwischen der HF-Empfangseinheit und der dazugehörigen HF-Sendeeinheit und eine Synchronisiereinrichtung, insbesondere eine Verzögerungsschaltung umfassen, die derart ausgebildet ist, daß die in den HF-Augangssignalen aller Baugruppen enthaltenen Bildinformationen untereinander synchronisierbar und gleichzeitig durch die gespeicherte Bildinformation ersetzbar sind.

## FIG.1

## FIG.2

**FIG.3**

13 14 15 16

20 43 44 18

19 22 23 24 25 26 17

21

27

43 44

27'

31 48

28 34

30 29 32 33

37 35 36

38 40

41 45

39

42

**FIG.4**

24

## FIG.5

## FIG.6